# EUROPEAN PATENT SPECIFICATION

(11) **EP 4 410 343 B1**
(45) Date of publication and mention of the grant of the patent: **29.04.2026**
(21) Application number: 22875199.6
(22) Date of filing: 30.09.2022
(51) Int. Cl.: A61M 16/06, A61M 16/20

(54) **EXHAUST ASSEMBLY, VENTILATION ASSEMBLY, FRAME ASSEMBLY, AND MASK SYSTEM**
ABGASANORDNUNG, BELÜFTUNGSANORDNUNG, RAHMENANORDNUNG UND MASKENSYSTEM
ENSEMBLE D'ÉCHAPPEMENT, ENSEMBLE DE VENTILATION, ENSEMBLE CHÂSSIS ET SYSTÈME DE MASQUE

(30) Priority: 30.09.2021 CN 202111165548
(43) Date of publication of application: 07.08.2024
(73) Proprietor: BMC Medical Co., Ltd., Shijingshan Beijing 100041 (CN)
(72) Inventor: WANG, Yajie, Beijing 100041 (CN); ZHOU, Mingzhao, Beijing 100041 (CN); ZHUANG, Zhi, Beijing 100041 (CN)
(74) Representative: Grünecker Patent- und Rechtsanwälte PartG mbB
(86) International application number: PCT/CN2022/123527
(87) International publication number: WO 2023/051819

(56) References cited:
- EP-B1- 3 429 667
- WO-A1-2007/045023
- WO-A1-2013/006899
- WO-A1-2013/170290
- WO-A1-2020/172708
- WO-A1-2020/188495
- WO-A1-2021/046605
- CN-A- 101 732 787
- CN-A- 109 999 303
- CN-A- 110 382 030
- CN-A- 112 654 386
- CN-A- 113 842 533
- CN-U- 216 985 990
- US-A1- 2017 128 687
- US-A1- 2021 113 788
- US-A1- 2021 244 905
- US-B2- 10 960 164

## Description

### CROSS REFERENCE TO RELATED APPLICATIONS

The present application claims the priority of Chinese patent application CN 202111165548.8 filed on September 30, 2021.

### TECHNICAL FIELD

The present disclosure relates to the technical field of treatment of respiratory-related diseases, in particular to an exhaust assembly, a ventilation assembly, a frame assembly, and a mask system.

### BACKGROUND

A Chronic Obstructive Pulmonary Disease (COPD) is a common chronic disease which may develop from chronic bronchitis and (or) emphysema characterized by airflow obstruction into cor pulmonale and respiratory failure. The above-mentioned respiratory diseases are usually treated by adopting Continuous Positive Airway Pressure (CPAP) treatment and Non invasive ventilation (NIV). Its principle is that a treatment device provides a CPAP to keep an airway open and prevent an upper airway from being obstructed. Even so, if a patient feels that the treatment device is uncomfortable, poor in usability, etc., the patient may refuse treatment.

A patient interface is an interface device worn on the face of a patient during ventilation treatment, and an existing common interface device is referred to as a mask. For example, breathable air is provided to an inlet of an airway, the air flows to be provided to the nose or mouth of the patient through the mask so as to be delivered to the airway of the patient. As required by treatment, the patient interface may form sealing, for example, the mask and facial region of the patient form sealing, so that gases are delivered to realize treatment under the condition that an ambient pressure is variable enough, for example, the pressure is a positive pressure of about twelve-centimeter H2O.

A mask system is an interface device worn on the face of a patient during ventilation treatment, air flows to be provided to the nose or mouth of a user through the mask system so as to be delivered to the airway of the patient. As required by treatment, the mask system may form sealing, for example, a full-face mask can cover the nose and mouth of the user and form sealing with the facial region of the user, thereby satisfying the requirement for opening the mouth to breathe; and the mask system is high in universality and better in Qi and blood parameters for treatment.

However, the full-face mask also has obvious defects, that is, the full-face mask is heavy, large in dead space and obvious in highlighting effect as a whole so as to be poorer in viewing field when being worn; and the full-face mask may press against the nasal bridge when being worn, which makes a user incapable of speaking or coughing, and therefore, the comfort level is poor. In addition, the state of an illness of a user suffering from claustrophobia may be aggravated. WO2020172708A1 discloses a vent housing of a vent structure comprising an inlet, an outlet, at least one exhaust gas orifice, and a membrane having a first end and a second end spaced apart, wherein the membrane has a moveable portion between the first end and the second end. WO2013006899A1 discloses a swivel elbow and connector assembly for a patient interface system comprising a ring configured to be sealingly secured in an aperture of the patient interface system, wherein the ring comprises a first side in an interior of the patient interface system and a second side at an exterior of the patient interface system when the ring is secured in the aperture, and the ring futher comprises a plurality of vents configured to permit flow of gases from the interior to the exterior of the patient interface system. US10960164B2 discloses a vent assembly for use with a respiratory mask of the type used in CPAP treatment comprising a porous disk portion that is attached to a biasing member such that the disk portion is maintained in a substantially sealed position against a main vent to minimize airflow through at least one side vent of the vent assembly. WO2013170290A1 discloses a swivel elbow and connector assembly for a patient interface system comprising a ring configured to be sealingly secured in an aperture of the patient interface and an elbow swivably secured in the ring. CN110382030A discloses a vent assembly for a respiratory pressure therapy (RPT) system comprising a vent housing having a first orifice configured to receive the flow of pressurized gas from the RPT device and the vent housing having a plurality of holes to discharge pressurized gas to atmosphere. WO2020188495A1 discloses a patient interface comprsing a plenum chamber, a seal-forming structure, a positioning and stabilizing structure, a plenum chamber insert configured to be positioned and retained within the plenum chamber, and a vent structure, wherein when the plenum chamber insert is positioned and retained within the plenum chamber, a radial channel is formed by the interior surface of the plenum chamber and the exterior surface of the plenum chamber insert such that gas is able to pass between a patient-proximal side of the plenum chamber insert and a patient-distal side of the plenum chamber insert via the radial channel during use. US20210113788A1 discloses interfaces for positive pressure therapy comprising a bias flow vent with geometries that help reduce and/or minimize draft and noise levl of the fluids exciting the vents. EP3429667B1 discloses a respiratory mask system for the delivery of respiratory therapy to a patient including a headgear comprising an integrally formed closed loop, wherein the closed loop comprises a yoke configured to connect to a patient interface, a pair of side arms configured to extend from a lateral rearward portion of the yoke, and in use, across a cheek and above an ear of a suer, and a top strap configured to extend between the pair of side arms, and in use, across the top of the user's head. EP20210244905A1 discloses a nasal seal, mask or an interface assembly comprising a seal body defining a breathing chamber, wherein a nasal port is provided in the seal body comprising a central portion straddled by a pair of lateral portions, the nasal port further comprises an upper edge defining an inwardly projecting portion within the central portion, and a lower edge defining an inwardly protection portion within the central portion. WO2021046605A1 discloses a patient interface system for delivery of respiratory therapy to a patient comprising a nasal portion including a nasal chamber and a nasal sealing portion adapted to form a seal with the patient's face surrounding the entrance to a patient's nares, wherein the nasal chamber comprises an opening; an opening, an inlet conduit connected to the nasal portion to deliver the pressurized, breathable gas to the nasal chamber.

### SUMMARY

The invention is defined by the appended claims. Subject-matter referred as embodiments and/ or disclosures which does not fall under the scope of the claims is not part of the invention.

The present disclosure provides an exhaust assembly, a ventilation assembly, a frame assembly, and a mask system to solve the above-mentioned technical problems.

According to a first aspect of the present disclosure, the present disclosure provides an exhaust assembly for a mask system, including a first main body, the first main body including an intake hole and a side wall disposed around the circumferential direction of the intake hole, and exhaust holes being disposed in the side wall; a gas entering the intake hole being configured to be delivered to a wearer wearing the mask system, and the gas exhaled by the wearer being configured to be exhausted from the exhaust holes; and
inner hole diameters of the exhaust holes are smaller than or greater than outer hole diameters of the exhaust holes
an included angle a being formed between a normal plane in an intake direction in the intake hole and the side wall.

In an implementation, the included angle a between the normal plane in the intake direction in the intake hole and the side wall is ten degrees to seventy degrees.

In an implementation, an included angle ß is formed between the inner side wall of the exhaust holes and the normal plane.

In an implementation, the included angle ß between the inner side wall of the exhaust holes and the normal plane is eighty degrees to one hundred degrees.

In an implementation, the average thickness of the side wall is zero point five millimeters to two point zero millimeters.

In an implementation, the exhaust holes are constructed to ensure that a direction in which the gas enters the exhaust holes and a direction in which the gas is exhausted from the exhaust holes are parallel and non-collinear.

In an implementation, the exhaust holes are strip-shaped, round, elliptic or oblong.

In an implementation, the exhaust holes are annularly disposed in the circumferential direction of the intake hole.

In an implementation, the exhaust holes are distributed in a circle or ellipse concentric with the intake hole.

In an implementation, the exhaust holes are formed by up-and-down collision puncturing of molds.

In an implementation, the first main body is constructed on a frame assembly of the mask system, and forms a split structure or an integrated structure with the frame assembly of the mask system.

In an implementation, the first main body is constructed on a frame assembly and a cushion assembly of the mask system, and respectively forms an integrated structure with the frame assembly and the cushion assembly of the mask system.

In an implementation, the first main body is constructed on a cushion assembly of the mask system, and forms a split structure or an integrated structure with the frame assembly of the mask system.

According to a second aspect of the present disclosure, the present disclosure provides a ventilation assembly for a mask system, including an exhaust assembly and a second main body,
the exhaust assembly including a first main body, a safety member being disposed on the second main body, the safety member being constructed to enable an intake hole of the exhaust assembly to communicate with a gas device when a gas is introduced to the mask system, and enable the mask system to communicate with an environment when no gas is introduced to the mask system.

In an implementation, the exhaust assembly is the above-mentioned exhaust assembly.

In an implementation, the second main body and the exhaust assembly form the split structure, the exhaust assembly is constructed on a frame assembly of the mask system, and the second main body is constructed as a pipe body connected to the frame assembly.

In an implementation, the safety member includes a safety valve hole and a safety valve plate, the safety valve hole is connected to the intake hole, and the safety valve plate is constructed to enable the intake hole and the safety valve hole not to be opened or closed at the same time.

In an implementation, the safety valve plate is disposed in the intake hole and is rotatably connected to an inner wall of the intake hole, when the safety valve plate rotates to a first position, the safety valve hole is closed, and the intake hole communicates with the gas device; and when the safety valve plate rotates to a second position, the safety valve hole is opened, and the intake hole does not communicate with the gas device.

In an implementation, the first main body and the second main body form the split structure, the first main body is constructed on a frame assembly of the mask system, and the second main body is integrally constructed on a cushion assembly of the mask system.

In an implementation, the first main body and the second main body form the integrated structure, and the first main body and the second main body are constructed on a frame assembly or a cushion assembly of the mask system.

In an implementation, the first main body and the second main body form the split structure, the first main body is constructed on a frame assembly of the mask system, and the second main body is constructed as an elbow connected to the frame assembly.

In an implementation, the safety member includes a safety valve hole and a safety valve plate, the safety valve hole is connected to the elbow, and the safety valve plate is constructed to enable the intake hole and the safety valve hole not to be opened or closed at the same time.

In an implementation, the safety valve plate is disposed in the elbow and is rotatably connected to an inner wall of the elbow, when the safety valve plate rotates to a first position, the safety valve hole is closed, and the elbow is opened, so that the intake hole communicates with the gas device; and when the safety valve plate rotates to a second position, the safety valve hole is opened, and the elbow is closed, so that the intake hole does not communicate with the gas device.

According to a third aspect of the present disclosure, the present disclosure provides a frame assembly, including a frame on which the above-mentioned exhaust assembly and/or the above-mentioned ventilation assembly are constructed.

In an implementation, a cushion connecting part is disposed on the frame, and the cushion connecting part is matched with and connected or stuck to an oronasal cushion of the mask system.

In an implementation, the cushion connecting part is constructed as a boss on the inner side of the frame, a buckling part is disposed on the cushion connecting part, a sealing part is disposed on an inner wall of an air inlet of the exhaust assembly, and the cushion connecting part is matched with and connected to the sealing part.

In an implementation, a first extension body and a second extension body extending obliquely and upwards are respectively disposed on two sides of the upper part of the frame, and the first extension body and the second extension body cover a part of the outer side of the oronasal cushion.

In an implementation, a first bone beam arm and a second bone beam arm are respectively disposed on the first extension body and the second extension body, and the first bone beam arm and the second bone beam arm are respectively connected to upper headbands in a headband of the mask system; and
the first bone beam arm and the second bone beam arm respectively extend in extension directions of the first extension body and the second extension body.

In an implementation, the first bone beam arm, the second bone beam arm and the frame are respectively formed, or
the first bone beam arm, the second bone beam arm and the frame are integrally formed.

In an implementation, the thickness of the first bone beam arm and the thickness of the second bone beam arm are both zero point six millimeters to one point five millimeters.

In an implementation, the first bone beam arm and the second bone beam arm are made of PC, PP or ABS and other thermoplastic materials.

In an implementation, a first bone beam arm connecting hole and a second bone beam arm connecting hole are respectively disposed in the first bone beam arm and the second bone beam arm, and the first bone beam arm connecting hole and the second bone beam arm connecting hole are respectively connected to the upper headbands in the headband of the mask system.

In an implementation, a first headband connecting hole and a second headband connecting hole are respectively disposed in the first extension body and the second extension body, and the first headband connecting hole and the second headband connecting hole are respectively connected to upper headbands in a headband of the mask system.

In an implementation, a first opening and a second opening are respectively disposed in the first extension body and the second extension body, and the first opening and the second opening respectively extend in extension directions of the first extension body and the second extension body.

In an implementation, a third headband connecting hole and a fourth headband connecting hole are respectively disposed in two sides of the lower part of the frame; and the third headband connecting hole and the fourth headband connecting hole are respectively connected to lower headbands in a headband of the mask system.

In an implementation, a first hasp and a second hasp are respectively disposed in the third headband connecting hole and the fourth headband connecting hole, and the lower headbands are connected to the third headband connecting hole and the fourth headband connecting hole through the first hasp and the second hasp.

According to a fourth aspect of the present disclosure, the present disclosure provides a mask system, including a cushion assembly, the cushion assembly including an oronasal cushion, the oronasal cushion including a nose structure and a mouth structure, a through cavity capable of receiving a pressure gas is formed in the nose structure and the mouth structure, the nose structure is adaptively fitted around nostrils of a user so as to seal; and the mouth structure is adaptively fitted around the mouth of the user so as to seal;
wherein the nose structure is constructed to have no clamping effect on nosewings of a patient when pressing against the nasal base of a wearer by means of a pressurized gas so as to seal.

In an implementation, the mask system also includes the exhaust assembly as mentioned above and/or the ventilation assembly as mentioned above.

In an implementation, the mask system also includes the frame assembly as mentioned above, and the frame assembly is detachably connected to the oronasal cushion.

In an implementation, the frame assembly is directly connected to an elbow, the elbow is connected to an air delivery pipe, and the air delivery pipe is configured to be connected to a pressure device; and
the internal diameter of the air delivery pipe is fifteen millimeters to twenty-two millimeters.

In an implementation, the frame is directly connected to a flexible hose, the flexible hose is connected to an air delivery pipe, and the air delivery pipe is configured to be connected to a pressure device; and the internal diameter of the flexible hose is twelve to fifteen millimeters, and the internal diameter of the air delivery pipe is fifteen to twenty-two millimeters.

In an implementation, the flexible hose is an elastic pipe.

In an implementation, a connector is disposed on the frame, and the connector is rotatably connected to the flexible hose.

In an implementation, the mask system also includes a headband, the headband is respectively connected to two sides of the frame, and the headband is constructed to be fixed around the head of the user in response to the mask system being worn by the user.

In an implementation, the headband includes:
upper headbands respectively connected to a first headband connecting hole and a second headband connecting hole of the frame assembly, and the upper headbands being constructed to extend to upsides of ears along the cheeks of the user in response to the headband being worn by the user so as to adjust a pressure of the oronasal cushion on the nose of the user; and
lower headbands respectively connected to a third headband connecting hole and a fourth headband connecting hole of the frame assembly, and the lower headbands being constructed to extend to the back of the head of the user along two sides of a chin of the user in response to the headband being worn by the user.

In an implementation, the upper headbands and the lower headbands form an integer on the back of the head of the wearer.

In an implementation, the headband also includes a top headband, and the top headband includes a first end band and a second end band respectively connected to the upper headbands, wherein a connecting piece is disposed on one of the first end band and the second end band, a matching member is disposed on the other one of the first end band and the second end band, and the matching member is connected to the connecting piece, so that the first end band and the second end band are connected on the top of the head of the wearer.

Compared with the prior art, the present disclosure has the advantages that the mask system can satisfy the requirement for opening the mouth to breathe by respectively covering the nostrils and the mouth of the a user with a nose structure and a mouth structure when being worn, thereby having the advantage of good clinic Qi and blood parameters; at the same time, the mask system seals around the nostrils of the user and avoids pressing against the nasal bridge or two sides of the nosewings, and therefore, the structure is smaller and lighter, the viewing field is good, and then, the comfort level is improved; and compared with a full-face mask, a nasal mask in the present disclosure is not prone to claustrophobia and better in people adaptability. Therefore, the mask system in the present disclosure not only can overcome defects of poor comfort level and poor people adaptability of the existing full-face mask due to easily pressing against the nasal bridge when being worn, but also can remain the advantage of the full-face mask capable of satisfying the requirement for opening the mouth to breathe and good in clinic Qi and blood parameters, thereby improving the treatment effect and the treatment compliance.

### BRIEF DESCRIPTION OF THE DRAWINGS

The present disclosure will be described in more detail hereinafter based on embodiments and with reference to accompanying drawings.
FIG. lisa schematic view of a basic structure of a human nose;
FIG. 2 is a schematic view (observed from the rear side) of a three-dimensional structure of an oronasal cushion of a mask system in embodiment 1 of the present disclosure;
FIG. 3 is a schematic view (observed from the front side) of the three-dimensional structure of the oronasal cushion of the mask system in embodiment 1 of the present disclosure;
FIG. 4 is a main view of the oronasal cushion of the mask system in embodiment 1 of the present disclosure;
FIG. 5a is a schematic view of a positional relationship between the oronasal cushion of the mask system in embodiment 1 of the present disclosure and the nose of a user, in which a thick dash line shows a position when a middle part deforms, and thin dash lines show boundaries between the middle part and each of two nose side parts;
FIG. 5b is a schematic view of wearing of the oronasal cushion of the mask system in embodiment 1 of the present disclosure, in which it is shown that the middle part has expansively deformed to cling around nostrils of a user, and thin dash lines show a position when the middle part does not deform;
FIG. 6a is a top view of the oronasal cushion of the mask system in embodiment 1 of the present disclosure, in which dash lines show boundaries between the middle part and each of two nose side parts;
FIG. 6b is a sectional view of part C-C in FIG. 6a;
FIG. 6c is a sectional view of part D-D in FIG. 6a;
FIG. 7 is a sectional view of the oronasal cushion of the mask system in embodiment 1 of the present disclosure, in which dash lines show the nose of a user;
FIG. 8 is a top view of an oronasal cushion of a mask system in another embodiment of the present disclosure, in which dash lines show boundaries between a middle part and each of two nose side parts;
**FIG.** 9 is a main view of the oronasal cushion of the mask system in embodiment 1 of the present disclosure, in which a chin is shown, and dash lines show boundaries between each of two mouth side parts and the chin;
FIG. 10 is a sectional view of part A-A in FIG. 9;
FIG. 11 is a sectional view of part B-B in FIG. 9;
FIG. 12 is a schematic view (observed from the rear side) of a three-dimensional structure of an oronasal cushion of a mask system in embodiment 2 of the present disclosure;
FIG. 13 is a schematic view (observed from the front side) of the three-dimensional structure of the oronasal cushion of the mask system in embodiment 2 of the present disclosure;
FIG. 14 is a main view (observed from the rear side) of the oronasal cushion of the mask system in embodiment 2 of the present disclosure;
FIG. 15 is a sectional view of part E-E in FIG. 14;
FIG. 16 is a schematic view of a three-dimensional structure of a mask system in an embodiment of the present disclosure;
FIG. 17 is a schematic view of a three-dimensional structure of a frame assembly shown in FIG. 17;
FIG. 18 is a main view of a frame shown in FIG. 17;
FIG. 19 is a sectional view of part F-F in FIG. 17, in which an arrow shows an intake direction of a pressure airflow in an intake hole, and dash lines show a normal plane in the intake direction of the pressure airflow in the intake hole;
FIG. 20 is a sectional view of exhaust of the frame shown in FIG. 17, in which an arrow shows that a gas flows out of exhaust holes;
FIG. 21 is a lateral view of the frame shown in FIG. 17, in which an arrow shows that a gas flows out of exhaust holes;
FIG. 22 is a schematic view of a three-dimensional structure of the frame shown in FIG. 17, in which an arrow shows that a gas flows out of exhaust holes;
FIG. 23 is a sectional view of a frame assembly shown in FIG. 16;
FIG. 24 is a rear view of the frame assembly shown in FIG. 16;
FIG. 25 is a schematic structural view of connection between the frame assembly shown in FIG. 16 and a ventilation pipe;
FIG. 26 is a schematic view of a three-dimensional structure of a frame in another embodiment of the present disclosure;
FIG. 27 is a schematic view of a three-dimensional structure of a mask system in another embodiment of the present disclosure;
FIG. 28 is a schematic view of a three-dimensional structure of a mask system in further embodiment of the present disclosure;
FIG. 29 is a schematic view of a three-dimensional structure of a mask system in yet further embodiment of the present disclosure;
FIG. 30 is a schematic view of a three-dimensional structure of a mask system in further another embodiment of the present disclosure;
**FIG. 31** is a schematic view of a three-dimensional structure of a mask system in yet further another embodiment of the present disclosure; and
FIG. 32 is a schematic view of wearing of a full-face mask in the prior art.

Reference numerals in the accompanying drawing:
1-oronasal cushion;
2-nose structure; 21-nose opening; 22-nose pad part; 23-middle part; 24-two nose side parts; 25-local thickened part;
3-mouth structure; 31-mouth opening; 32-mouth pad part;
34-two mouth side parts; 341-face contact region; 342-face supporting region; 343-mouth transition region;
35-chin; 351-chin contact region; 351-chin supporting region; 353-chin transition region;
4-reinforced structure;41-air inlet; 42-supporting transition region; 43-connecting part; 411-sealing part;
5-oronasal transition part; 6-cavity;
NI-nostril; N2-nosewing; N3-apex of nose; N4-nasal bridge; N5-nasal depression; F-cheek;
400-mask system;
50-frame assembly; 51-frame; 52-first headband connecting hole; 53-second headband connecting hole; 54-third headband connecting hole; 55-fourth headband connecting hole; 56-hole inner side; 57-hole outer side;
511-first extension body; 512-second extension body; 513-first opening; 514-second opening;
500-elbow; 600-elbow connector;
60-exhaust assembly; 61-exhaust hole; 62-intake hole; 63-side wall;
70-safety member; 71,501-safety valve hole; 72,502-safety valve plate; 73-pipe connecting part;
90-headband; 91,92-upper headband; 94,95-lower headband; 93-top headband; 96-first hasp; 97-second hasp;
80-flexible hose; 81-first connecting end; 82-second connecting end; 83-connector;
700-first bone beam arm; 701-second bone beam arm; 703-first bone beam arm connecting hole; and 704-second bone beam arm connecting hole.

### DETAILED DESCRIPTION OF THE EMBODIMENTS

The present disclosure will be further described below in conjunction with the accompanying drawings.

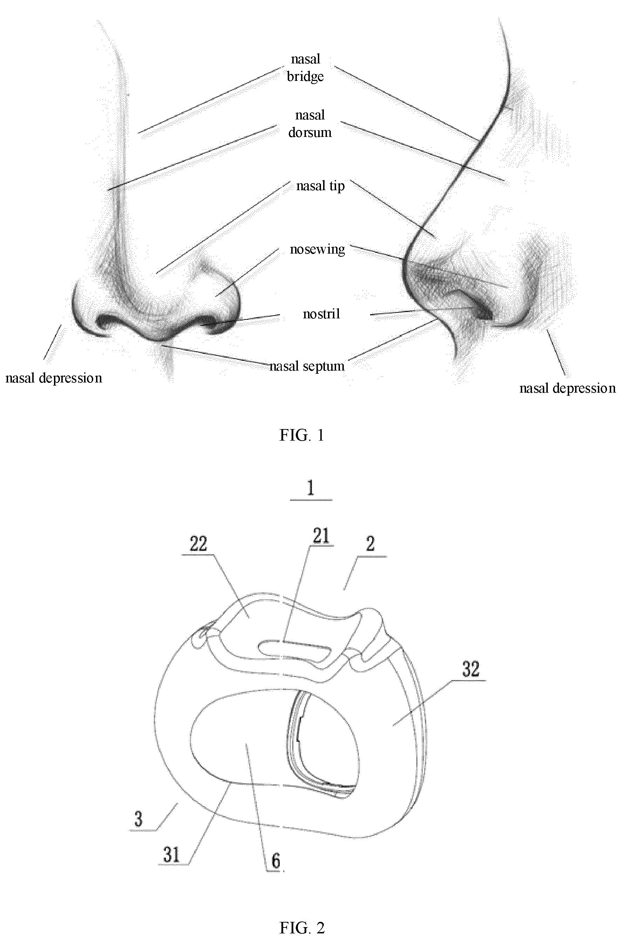

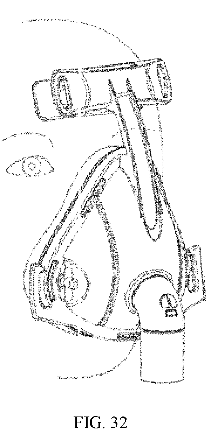

As shown in FIG. 1, nose features of a common user are shown. Compared with an existing full-face mask shown in FIG. 32, a mask system in the present disclosure cannot cover the nasal bridge of a user or press against a sensitive nasal bridge region of the user, so that the comfortness is greatly improved; in addition, the mask system is smaller and lighter and better in viewing field of the user; and the mask system is better in people adaptability, less prone to claustrophobia, and more comfortable to wear.

Specifically, the mask system in the present disclosure includes an exhaust assembly 60, a ventilation assembly, a cushion assembly, a frame assembly 50, and a headband 90. The cushion assembly includes an oronasal cushion 1, and the frame assembly 50 is detachably connected to the oronasal cushion 1. A headband 90 is connected to the frame assembly 50, and the headband 90 is constructed to be fixed around the head of a user in response to a mask system 400 being worn by the user.

The exhaust assembly 60 and the ventilation assembly can form a split structure or an integrated structure. In addition, any one or both of the exhaust assembly 60 and the ventilation assembly can be integrally formed with the frame assembly; or any one or both of the exhaust assembly 60 and the ventilation assembly can be integrally formed with the cushion assembly. Therefore, when all of the above-mentioned components form an integrated structure, all the components can share a part of structure.

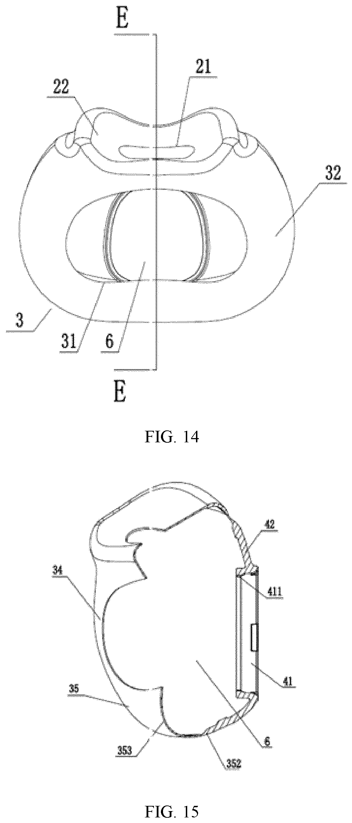

The oronasal cushion 1 in the present disclosure will be described in detail below in conjunction with FIG. 2 to FIG. 15 and all the embodiments.

The oronasal cushion 1 includes a nose structure 2 and a mouth structure 3 which are connected, and a through cavity 5 is formed inside the nose structure 2 and the mouth structure 3. The cavity 6 can receive a pressure gas from a pressure device and respectively deliver the pressure gas to the nose and mouth of a user through the nose structure 2 and the mouth structure 3. Therefore, when the cavity receives the pressure gas, an application range of a pressure is four to forty hectopascals, and thus, it is necessary to dispose the mouth structure 3 to accommodate the mouth of the user and share a part of face contact and support functions.

The oronasal cushion 1 in the present disclosure will be described below with specific embodiments.

### Embodiment 1

With reference to FIG. 2, the nose structure 2 includes a nose opening 21 communicating with the cavity 6 and a nose pad part 22 surrounding the nose opening 21. The nose opening 21 is constructed to surround lower sides of nostrils of a user in response to the oronasal cushion 1 being worn by the user, and is adaptively fitted around the nostrils of the user through the nose pad part 22 so as to seal.

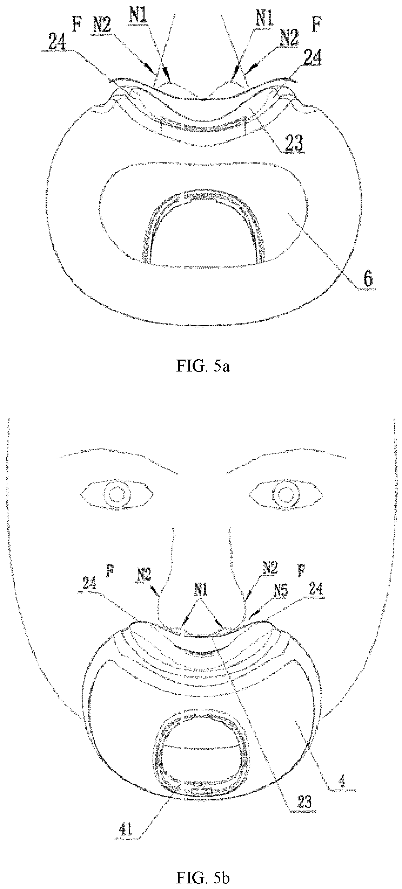

Specifically, with reference to FIG. 5, the nose pad part 22 includes a middle part 23 arranged around the nose opening 21 and two nose side parts 24 disposed on two sides of the middle part 23, and the middle part 23 deforms in response to the increase of a pressure in the cavity 6 so as to be fitted around the nostrils of the user. The thickness of the middle part 23 may be zero point two to one point zero millimeters, that is, a region where the middle part 23 is located is a thin film region, and therefore, the middle part 23 may expansively deform at a pressure to cling around the nostrils of the user. Then, even if there are individual differences in noses of users, it can be ensured that the middle part 23 can be adaptively fitted around the nostrils of the different users due to the characteristic of variability, and thus, the application range and sealing stability thereof are improved. In other words, the oronasal cushion 1 in the present disclosure is fitted around the nostrils of the user by means of expansive deformation of the middle part 23, and thus, it is unnecessary to consider individual differences in the noses of the users during design and manufacture. In other words, the nose structure 2 can be adaptively fitted around the nostrils of the user so as to seal, and the nose structure 2 is constructed to have no clamping effect on nosewings of a patient when pressing against the nasal base of a wearer by means of a pressurized gas so as to seal. Therefore, even if there are individual differences in the noses of the users, it can also ensure that the middle part 23 can be adaptively fitted around the nostrils of the user due to the characteristic of deformability of a thin film, thereby improving the sealing properties of contact and the stability of connection between the oronasal cushion 1 and the face of the user.

More specifically, the middle part 23 is sunken towards the inside of the cavity 6, that is, the middle part 23 is slightly lower than the two nose side parts 24 (as shown in FIG. 5a). Therefore, when the middle part 23 expands under the action of the pressure in the cavity 6, its height will be close to the heights of the two nose side parts 24, as shown in FIG. 5b. After the middle part 23 expands, the original small arc ring becomes a large arc ring, and the middle part 23 and the two nose side parts 24 are even almost located in the same plane, which can ensure that a part, contact with the face of the user, of the oronasal cushion 1 supports against the lower side of the nose of the user without wrapping two sides of the nosewings of the user. Therefore, the oronasal cushion 1 in the present disclosure can ensure the sealing stability without clamping the nosewings of the user, the oronasal cushion 1 in the present disclosure does not need to be strictly adapted to the width (a distance between the nosewings on the two sides, as shown in FIG. 1) of the nose of the user, and thus, the people adaptability can be improved.

As shown in FIG. 5a, the middle part 23 is slightly lower than the two nose side parts 24 when not deforming, and may be located on a position shown by a thick dash line in FIG. 5a when expansively deforming. It can be clearly shown in FIG. 5b that the middle part 23 bulges upwards after deforming, thereby sealing around nostrils N1 of the user, but cannot wrap nosewings N2 and nasal depressions N5 of the user.

In addition, the oronasal cushion 1 in the present disclosure does not clamp the nose of the user, therefore, the contact area between the nasal cushion and the nose of the user can be reduced, and the wearing comfort level can be improved.

Preferably, a front end 25 of the middle part 23 cannot exceed an apex of the nose N3 of the user, in this way, a nasal bridge N4 of the user cannot be pressed during wearing, the wearing comfortness can be further improved, and at the same time, a better viewing field is brought for the user during wearing.

The two nose side parts 24 mainly play a support role so as to have greater stresses than the middle part 23, and thus, the thickness of the two nose side parts 24 may be set to be greater than the thickness of the middle part 23. For example, the thickness of the two nose side parts 24 may be zero point six to one point five millimeters, and preferably zero point eight to one point two millimeters, which can ensure that their rigidity is higher than the rigidity of the middle part 23.

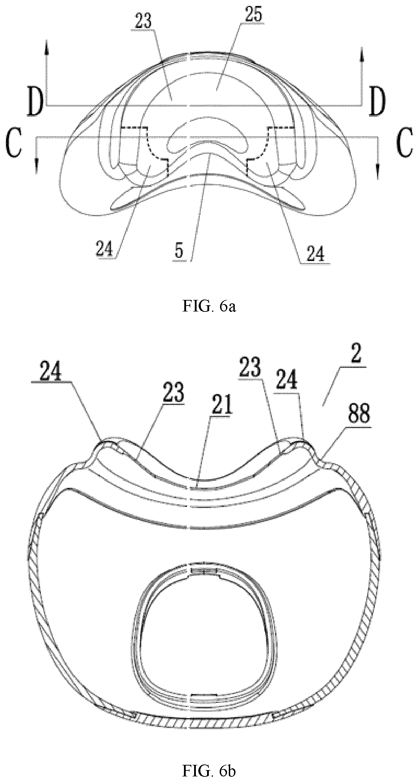

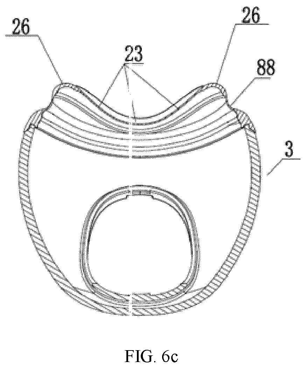

In an optional implementation, the two nose side parts 24 respectively extend at two sides of the middle part 23, as shown in FIG. 6a, FIG. 6b, and FIG. 6c, wherein dash lines in FIG. 6a show boundaries between each of two nose side parts 24 and the middle part 23, that is, regions where the two nose side parts 24 only extend within a local small range close to a wearing side of the face. In the implementation, when the oronasal cushion 1 is worn, the two nose side parts 24 are in contact with the face of the user first, and are closer to muscles of cheeks F of the user (as shown in FIG. 5a and FIG. 5b), the user does not obviously feel the stresses applied to the two nose side parts 24, and therefore, the two nose side parts 24 can effectively distribute a facial pressure generated when the oronasal cushion 1 is worn to improve the wearing comfort level. In addition, since the two nose side parts 24 are close to the face of the user, at the moment, the nose sealing stability of the oronasal cushion 1 depends on supporting forces of the two nose side parts 24 and an expansion force of the middle part 23. In the implementation, the regions where the two side parts 24 only extend within the local small range close to the wearing side of the face.

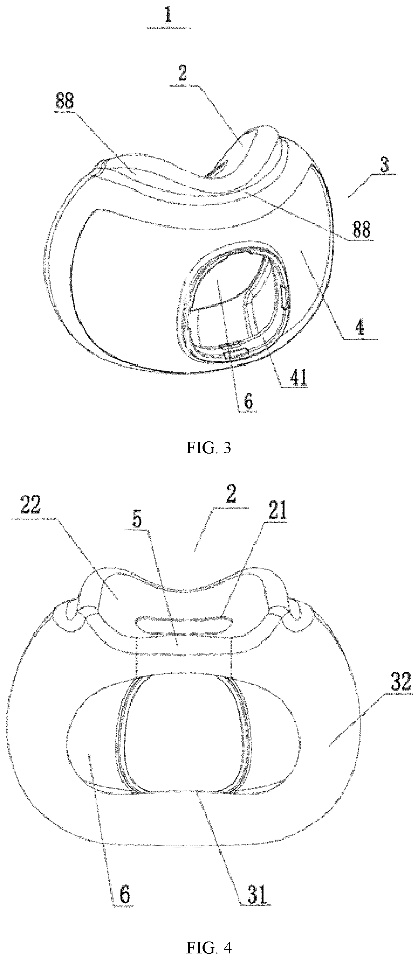

In the present embodiment, an oronasal connecting part 88 is disposed between two sides of the nose structure 2 and the mouth structure 3 and between front sides of the nose structure 2 and the mouth structure 3, as shown in FIG. 3, FIG. 6b, FIG. 6c, and FIG. 7, the oronasal connecting part 88 has a variable thickness. Specifically, the thickness of the oronasal cushion 88 between each of the two nose side parts 24 and the mouth structure 3 is generally equal to or greater than the thickness of the oronasal connecting part 88 of the two nose side parts 24.

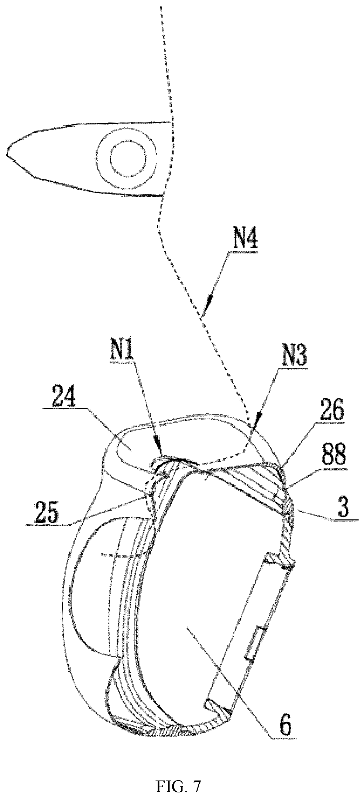

As shown in FIG. 6b and FIG. 7, the middle part 23 includes a middle local region 26, and the middle local region 26 is basically located on an edge zone, corresponding to the nose of the user and not easy to contact, on the nose structure 2. In the present embodiment, the thickness of the middle local region 26 is slightly greater than the thickness of the middle part 23, for example, it may be zero point two millimeters to one point two millimeters, and preferably zero point eight millimeters to zero point nine millimeters, and the middle local region 26 mainly plays a role in keeping an approximate shape of the thin film region of the middle part 23 during uninflation.

In addition, the middle local region 26 may also have the same thickness as the middle part 23 to ensure that there is no great impact on use.

A transition thickness of the oronasal connecting part 88 between the middle part 23 of the nose structure 2 and the mouth structure 3 may also be set as a thickness of a thin film, with reference to FIG. 6b and FIG. 7. The thickness of the oronasal connecting part 88 may be set to be zero point two millimeters to zero point six millimeters, and preferably zero point three millimeters to zero point five millimeters, and the oronasal connecting part 88 can better provide supplement for sealing a nose region during inflation.

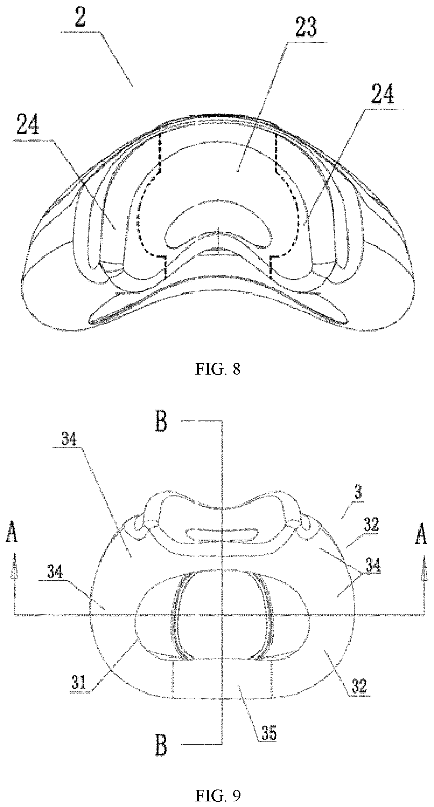

In another optional implementation, the two nose side parts 24 extend at two sides of the middle part 23 to reach the front end of the outer side of the middle part 23 so as to increase the area of a supporting region, as shown in FIG. 8, in which dash lines show the boundaries between each of two nose side parts 24 and the middle part 23. In the implementation, the two nose side parts 24 are larger in area, thereby providing better support. Therefore, at the moment, the nose sealing stability of the oronasal cushion 1 mainly depends on the supporting forces of the two nose side parts 24. In the implementation, the region where the two side parts 24 are located extends within a large range to reach the front side.

As mentioned above, the middle part 23 is thinner and is a thin film region. Therefore, optionally, the middle part 23 may be constructed as a structure with a uniform thickness. Further optionally, the middle part 23 is provided with one or more local thickening parts 25 so as to thicken local regions.

The number of the nose opening 21 may be set as required. In the present embodiment, one nose opening 21 is shown, and a plurality of nose openings 21 may also be disposed as required, for example, two nose openings 21 are disposed to respectively correspond to the two nostrils of the user, and respectively surround the lower sides of the corresponding nostrils of the user during wearing.

The oronasal cushion 1 also includes an oronasal transition part 5 disposed between the nose structure 2 and the mouth structure 3, and the oronasal transition part 5 is fitted to a lip region of the user in response to the oronasal cushion 1 being worn by the user. The oronasal transition part 5 is constructed to be of a concave shape in a direction away from the upper lip. Since the oronasal transition part 5 corresponds to a stress sensitive region on the face of the user, the thickness of the oronasal transition part 5 may be set to be the same or similar to the thickness of the middle part 23, that is, a region where the oronasal transition part 5 is located is also a thin film region to ensure that a pressing force in the lip region of the user is small enough during wearing, so that the wearing comfortness is improved.

In the implementations shown in FIG. 5a and FIG. 5b, the transition between each of the two nose side parts 24 to the oronasal transition part 5 trends to be of the concave shape in the direction away from the upper lip, in this way, the two nose side parts 24 can be in contact with the nasal depressions N5 on the face first. Therefore, when a mask is worn, the two nose side parts 24 are important supporting points for the face F, and have higher stresses than the sensitive middle part 23 and oronasal transition part 5 on the nose. In addition, the two nose side parts 24 are supported near the nasal depression N5, and are closer to the muscles on the cheeks F (shown in FIG. 5-1 of FIG. 5), and therefore, the user does not obviously feel the stress. In this way, a pressure generated when the cushion is worn on the face can be effectively distributed, and the wearing comfort level is improved.

The mouth structure 3 will be described in detail as below.

With reference to FIG. 4, the mouth structure 3 includes an mouth opening 31 communicating with the cavity and a mouth pad part 32 disposed around the mouth opening 31; the mouth opening 31 is constructed to accommodate the mouth of the user in response to the oronasal cushion 1 being worn by the user; and the mouth pad part 32 is constructed to be fitted to the face of the user in response to the oronasal cushion 1 being worn by the user. Therefore, the mouth structure 3 is in a tendency as a whole that the middle part where the mouth opening 31 is located is sunken towards the inside of the cavity 6, and two side parts where the mouth pad part 32 is located are raised towards the outer side, thereby ensuring that there may be a larger contact area between the mouth pad part 32 and the face of the user to ensure a sealing effect.

A radial section of the mouth opening 31 is quasi-elliptic or elliptic. During wearing, the mouth pad part 32 seals the mouth of the user.

As shown in FIG. 9, the mouth pad part 32 includes two mouth side parts 34 and a chin 35, and dash lines show boundaries between each of the mouth side parts 34 and the chin 35. The oronasal transition part 5 and the chin 35 are respectively connected to upper and lower sides of the two mouth side parts 34; and the two mouth side parts 34 are constructed to be fitted to the face of the user in response to the oronasal cushion 1 being worn by the user, and the chin 35 is constructed to be fitted to the chin 35 of the user in response to the oronasal cushion 1 being worn by the user.

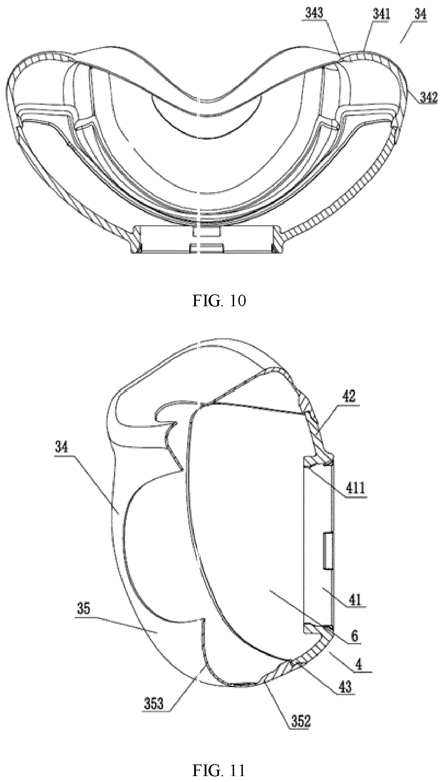

As shown in FIG. 10, the two mouth side parts 34 include a face contact region 341 surrounding the mouth opening 31, a face supporting region 342 smoothly connected to the face contact region 341 and extending to the front side of the mouth structure 3, and a mouth transition region 343 surrounding the mouth opening 31; and the mouth transition region 343 is smoothly connected to the face contact region 341 and the face supporting region 342.

Preferably, the thickness of the mouth transition region 343 is smaller than the thickness of the face supporting region 342, and the thickness of the face contact region 341 is smaller than or equal to the thickness of the face supporting region 342. The face supporting region 342 serves as a main stress supporting part when the oronasal cushion 1 is worn to ensure sealing stability with the face of the user, thereby needing to have certain rigidity. Therefore, the thickness of the face supporting region 342 may be one point two to two point five millimeters, and preferably about one point five millimeters.

In addition, the face contact region 341 is close to the face of a patient, and therefore, it can have the same thickness as the face supporting region 342. The mouth transition region 343 may adopt a smaller thickness, such as zero point three to zero point six millimeters, and a region where the mouth transition region 343 is located may be a thin film region. Or the mouth transition region 343 may also adopt other thickness smaller than the thickness of the face supporting region 342.

The chin 35 includes a chin contact region 351 surrounding the mouth opening 31, a chin transition region 353 smoothly connected to the chin contact region 351, and a chin supporting region 352 connected to the chin transition region 353 by means of smooth transition. The area of the chin supporting region 352 is very small, even as small as almost zero, at the moment, the chin transition region 353 is larger in area, and can directly extend to the front side of the oronasal cushion 1.

The chin contact region 351 may be smaller in thickness to form a thin film region. For example, its thickness may be zero point two millimeters to zero point eight millimeters, and preferably zero point two millimeters to zero point five millimeters.

Since the chin transition region 353 corresponds to the chin (jaw) of the user, i.e., a stress-sensitive part, the thickness d4 of the chin transition region 353 is the same as or similar to the thickness d5 of the chin contact part 351, so that the stress on the chin of the user during wearing is reduced, the wearing comfort level is improved.

In the present embodiment, the oronasal cushion 1 also includes a reinforced structure 4 located on the front side of the mouth structure 3 and connected to the mouth pad part 32 of the mouth structure 3, an air inlet 41 communicating with the cavity is disposed in the reinforced structure 4, a sealing part 411 is disposed on the air inlet 41, the air inlet 41 is detachably connected to the frame assembly 50 by the sealing part 411, and a pressure gas can be introduced to the cavity through the air inlet 41.

Optionally, the reinforced structure 4 is integrally molded with the mouth structure 3.

Optionally, the reinforced structure 4 and the mouth structure 3 are respectively made of different materials and are connected by a connecting part 43 on the reinforced structure 4. The connecting part 43 may be a mechanical connecting part or a chemical binder layer.

A part located between the air inlet 41 and the connecting part 43 of the reinforced structure 4 is a supporting transition region 42, and the thickness of the supporting transition region 42 is usually zero point eight millimeters to two point five millimeters, and is preferably one point two millimeters to one point eight millimeters. In this way, not only can the strength of the reinforced structure 4 be ensured, but also its portability can be ensured.

The reinforced structure 4 may be made of a plastic material PC or PP, and may also made of other thermoplastic materials such as high-transparency acrylics and ABS. Preferably, the reinforced structure 4 is made of transparent PC.

The nose structure 2 and the mouth structure 3 are made of silicon rubber or one of materials such as foam, thermoplastic elastomer, thermosetting material, foam, resin, and textiles.

When the nose structure 2 and the mouth structure 3 may be both made of silicon rubber, silicon rubber of which the shore hardness is thirty to forty is preferred.

### Embodiment 2

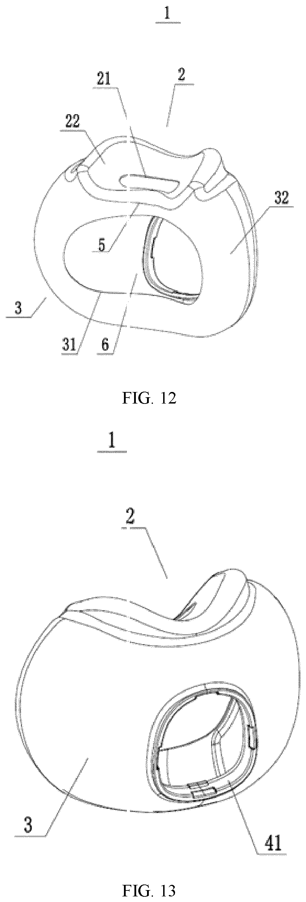

On the basis of above-mentioned embodiment 1, the present disclosure provides a variant embodiment, i.e., embodiment 2, as shown in FIG. 12 to FIG. 15. Differences from the aforementioned embodiment will be only described hereinafter, and the similarities thereof are no longer repeated.

The present embodiment differs from embodiment 1 in that the reinforced structure 4 is made of the same material as the mouth structure 3 in the original text, and they form an integer. The nose structure 2 may also be made of the same material as the reinforced structure 4 and the mouth structure 3, that is, the oronasal cushion 1 is made of the same material as a whole.

For example, the oronasal cushion 1 may be made of one of materials such as silicon rubber, foam, thermoplastic elastomer, thermosetting material, foam, resin, and textiles.

When the oronasal cushion 1 may be made of silicon rubber, silicon rubber of which the shore hardness is thirty to forty is preferred.

### Embodiment 3

On the basis of the above-mentioned embodiments, the present disclosure provides a variant embodiment, i.e., embodiment 3. Differences from the aforementioned embodiments will be only described hereinafter, and the similarities thereof are no longer repeated.

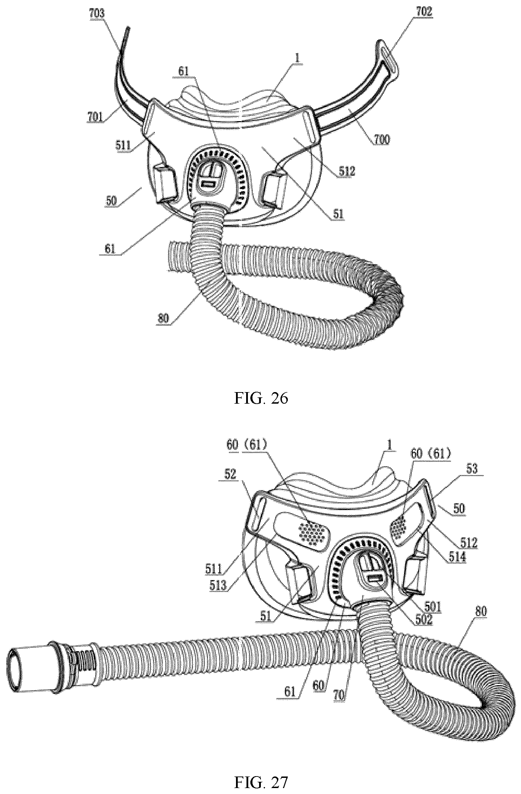

With reference to FIG. 27, the present embodiment differs from the above-mentioned embodiments in that exhaust assembly 60 is constructed on the upper part of the front side of the oronasal cushion 1. The exhaust assembly 60 includes a first main body (an exhaust part). The exhaust part may be a plurality of exhaust holes 61 disposed in the oronasal cushion 1. The first main body is integrally formed with the oronasal cushion 1.

The exhaust holes 61 are strip-shaped, oblong, round, elliptic or special-shaped. More specifically, projections of the exhaust holes 61 on a normal plane PI are strip-shaped, oblong, round, elliptic, rectangular or special-shaped (such as structures formed by two straight lines relatively parallel at least and at least one arc line connected to the two straight lines or sharp-angled special-shaped).

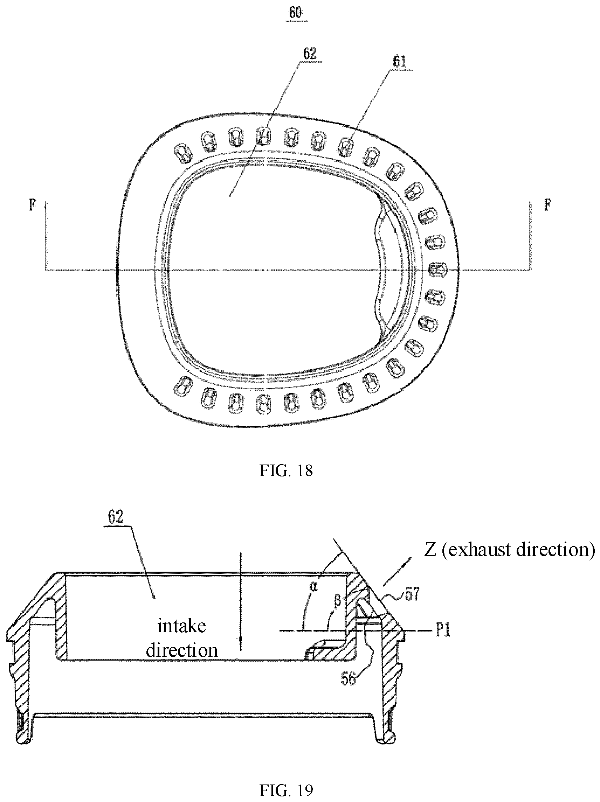

It should be noted that the normal plane PI is a plane perpendicular to an intake direction (a direction shown as an arrow in FIG. 19) of an intake hole 62 of each of the exhaust assembly 60. Therefore, it can be understood that if the intake direction of the intake hole 62 is an axial direction thereof, the normal plane PI is a radial direction of the intake hole 62.

Preferably, the exhaust holes 61 are strip-shaped, and may have larger single-hole area on a narrower surface in the exhaust direction. In this way, under the condition that the total exhaust is an expected constant value, there may be fewest holes, and the effect that a bed partner is followed by noise can also be reduced.

The plurality of exhaust holes 61 may be arranged in a round, an ellipse, array holes or a special shape, etc.

In addition, the inner hole area of the exhaust holes 61 is greater than or smaller than the outer hole area of the exhaust holes 61. Since the exhaust holes with the same internal and external diameters may cause louder noise, inner hole diameters of the exhaust holes 61 are set to be different from outer hole diameters of the exhaust holes 61, so that the exhaust noise is greatly lowered.

In addition, the exhaust holes 61 are arranged to diverge and are disposed to be diffused as much as possible, and at most six exhaust holes 61 are adjacent to each of the exhaust holes 61.

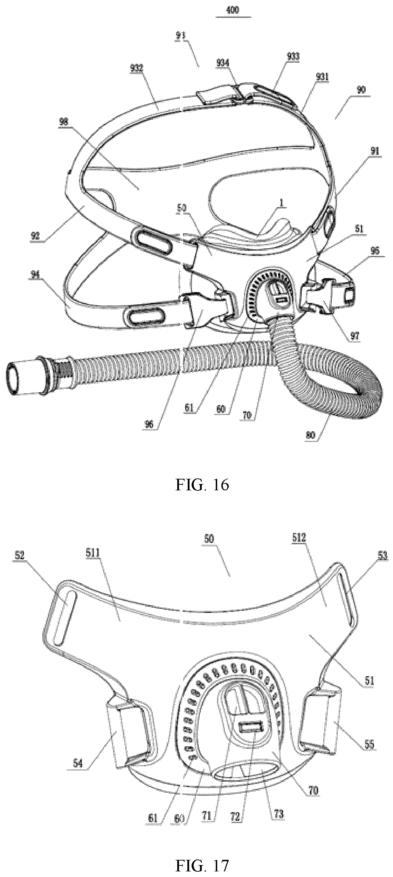

As shown in FIG. 16, the exhaust holes 61 are disposed in two sides of the air inlet 41, and are disposed to be away from the air inlet 41.

### Embodiment 4

On the basis of the above-mentioned embodiments, the present disclosure provides a variant embodiment, i.e., embodiment 4. Differences from the aforementioned embodiments will be only described hereinafter, and the similarities thereof are no longer repeated.

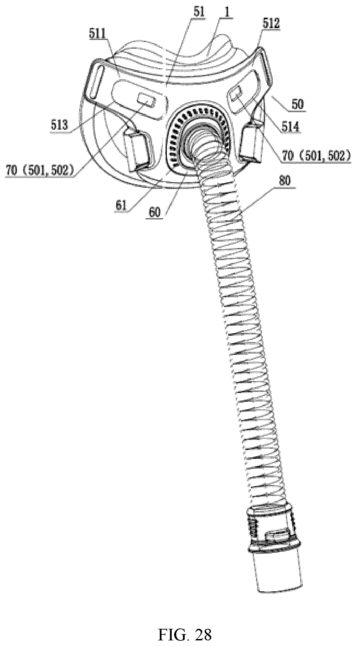

With reference to FIG. 28, the present embodiment differs from the above-mentioned embodiments in that the ventilation assembly is constructed on the oronasal cushion 1, and the ventilation assembly includes a second main body on which a safety member 70 is disposed. The second main body may be integrally formed with the oronasal cushion 1.

The safety member 70 includes a safety valve hole 501 and a safety valve plate 502 which are disposed on the upper part of the front side of the oronasal cushion 1. when a pressure device (unshown) introduces a pressure gas to the cavity 6 of the oronasal cushion 1 through the air inlet 41, the safety valve hole 501 is closed by the safety valve plate 502, and thus, the pressure gas can be input to a wearer through the oronasal cushion 1. On the contrary, when no pressure gas is introduced to the oronasal cushion 1, the safety valve hole 501 is opened by the safety valve plate 502, and thus, the oronasal cushion 1 communicates with the air through the safety valve hole, and the wearer can inhale the air outside the oronasal cushion 1 through the safety valve hole 501 to avoid the danger of asphyxia.

For the operation that the safety valve hole 501 is opened by the safety valve plate 502, the safety valve hole may be opened by adopting an existing safety valve plate, which is no longer repeated in the present disclosure.

### Embodiment 5

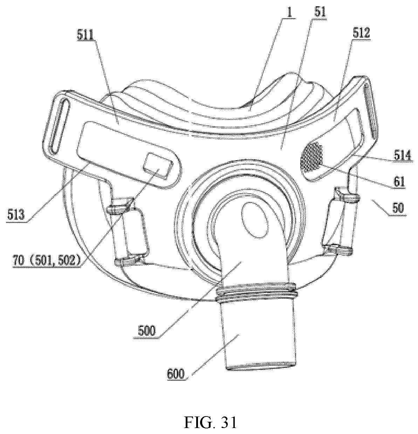

With reference to FIG. 31, on the basis of the above-mentioned embodiments, the present disclosure provides a variant embodiment, i.e., embodiment 5. Differences from the aforementioned embodiments will be only described hereinafter, and the similarities thereof are no longer repeated.

With reference to FIG. 31, the present embodiment differs from the above-mentioned embodiments in that the oronasal cushion 1 is provided with an exhaust assembly 60 (an exhaust holes 61) and a ventilation assembly (a safety member 70).

The exhaust holes 61 and the safety member 70 may be specifically disposed in a way described in embodiment 3 and embodiment 4 so as to be no longer repeated.

Specific structures of the frame assembly 50 and the headband 90 will be described in detail below in conjunction with FIG. 16 to FIG. 30. The oronasal cushion 1 in each of the above-mentioned embodiments may be combined with the frame assembly 50 in each of the embodiments described hereinafter.

As shown in FIG. 16, in an embodiment, the frame assembly 50 includes a frame 51, and the exhaust assembly 60 is integrally formed with the frame 51. The second main body is constructed as a pipe body, and the safety member 70 is connected to the frame 51. The frame 51 is detachably connected to the oronasal cushion 1 in a way such as clamping connection and threaded connection. The exhaust assembly 60 is connected to the frame 51 by buckling or other ways such as ultrasonic and chemical binding.

The headband 90 is respectively connected to two sides of the frame 51, and a way of connection between the headband 90 and the frame 51 will be described in detail hereinafter.

With reference to FIG. 18, the exhaust assembly 60 is connected to the frame 51, an intake hole 62 communicating with the cavity 6 is disposed in the exhaust assembly 60, the intake hole 62 is connected to a ventilation pipe 80, the intake hole 62 also communicates with the air inlet 41 of the oronasal cushion 1, and therefore, a pressure gas can be introduced to the cavity 6 through the intake hole 62.

The mask system 400 also includes an exhaust part. In some optional implementations, the exhaust part may be disposed on the oronasal cushion 1 as mentioned above.

In some preferred implementations, as shown in FIG. 17 to FIG. 23, the exhaust assembly 60 is constructed on the frame assembly 50.

Further, the exhaust assembly 60 is provided with an inclined side wall 63, and an included angle a formed between a normal plane PI in an intake direction of a pressure airflow in the intake hole 62 and an outer side wall of the exhaust assembly 60 is ten degrees to seventy degrees, and is preferably forty-five degrees to fifty-five degrees (as shown in FIG. 19). The exhaust part is disposed on the side wall 63. Specifically, the exhaust part includes a plurality of exhaust holes 61 passing through the exhaust assembly 60, and the exhaust holes 61 are constructed to be gradually reduced in area in a direction from the outer side to the inner side of the exhaust assembly 60. That is to say, the area of the exhaust holes 61 located on a hole inner side 56 on the inner side of the exhaust assembly 60 is smaller than the area of the exhaust holes 61 located on a hole outer side 57 on the outer side of the exhaust assembly 60, at the moment, the average thickness of the side wall 63 is zero point five millimeters to two point zero millimeters, and preferably, the average thickness of the side wall 63 is zero point nine millimeters to one point two millimeters. It can be understood that the thickness of the side wall 63 is basically the same under the condition of conventional wall thickness, that is, the wall thickness is usually not changed.

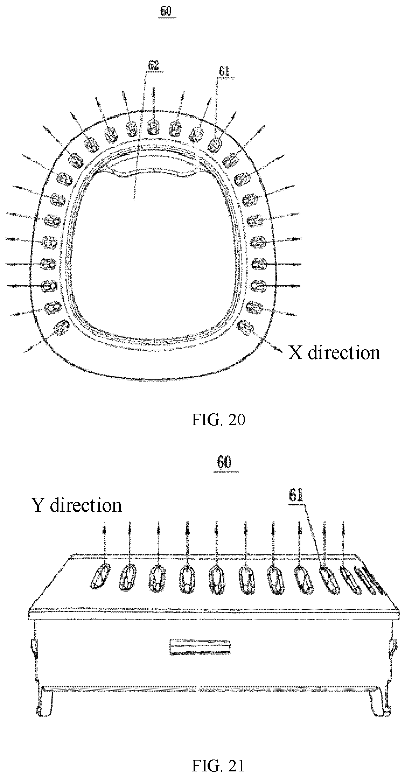

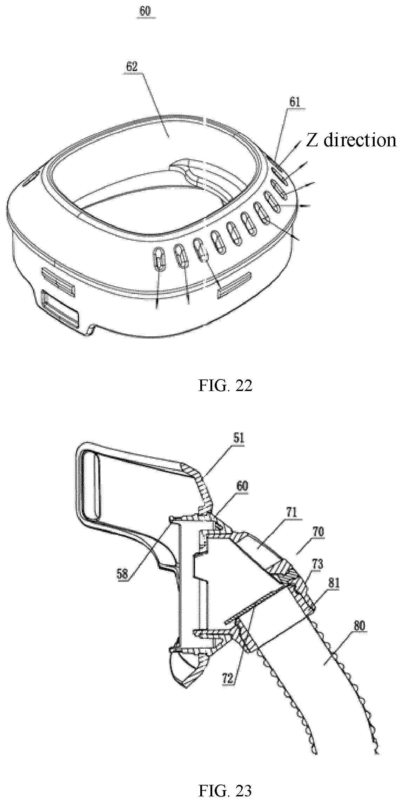

When observed from the normal plane, airflows in the above-mentioned exhaust holes 61 diverge from each other in X direction (a direction as shown by an arrow in FIG. 20); when observed from a mirror plane of the mask system, the airflows in the exhaust holes 61 diverge in Y direction (a direction as shown by an arrow in FIG. 21); and therefore, resultant forces diverge from each other to Z direction (a direction as shown by an arrow in FIG. 22). Such an arrangement has the advantages that the airflows can better diverge and can be rapidly attenuated after flowing out the exhaust holes 61, moreover, interference among the airflows from the exhaust holes 61 is not easy to occur, and therefore, a user side can achieve experience that the noise is lower, the impact energy of the airflows is lower, and the bed partner of the patient is not disturbed.

An included angle ß between the side wall of the exhaust holes 61 and the normal plane in the intake direction of the pressure airflow in the intake hole 62 is an acute angle. Preferably, the included angle is eighty-nine degrees to ninety degrees (as shown in FIG. 19).

In order to realize exhaust in Z direction, optionally, solutions of disposing a plurality of rows are on a mold or performing later laser drilling are adopted. However, these solutions are high in cost, incapable of ensuring the quality of holes, and louder in noise.

Preferably, the exhaust holes 61 are formed by up-and-down collision puncturing of molds. The exhaust holes 61 formed by up-and-down collision puncturing of the molds are better in technical moldability, lower in technical cost, capable of ensuring quality of the holes, and lower in noise during use.

The exhaust holes 61 are strip-shaped, round, elliptic or special-shaped, etc. More specifically, projections of the exhaust holes 61 on a normal plane PI are strip-shaped, oblong, round, elliptic, rectangular or special-shaped (such as structures formed by two straight lines relatively parallel at least and at least one arc line connected to the two straight lines or sharp-angled special-shaped).

Preferably, the exhaust holes 61 are strip-shaped, and may have larger single-hole area on a narrower surface in the exhaust direction. In this way, under the condition that the total exhaust is an expected constant value, there may be fewest holes, and the effect that a bed partner is followed by noise can also be reduced.

The exhaust holes 61 may be disposed in the circumferential direction of the intake hole 62. Specifically, the exhaust holes 61 may be disposed on a circle concentric with the intake hole 62 so as to achieve the best airflow divergency effect. Optionally, the exhaust holes 61 may also be arranged in an ellipse, array holes or a special shape on other parts on the frame 51. It can be understood that the "ellipse" mentioned in the present disclosure is an approximately-elliptic structure or a quasi-elliptic structure, is not limited as an ellipse formed according to a standard formula and curvature.

On the contrary, the exhaust holes 61 may also be constructed to be gradually reduced in area in a direction from the inner side to the outer side of the exhaust assembly 60. That is to say, the area of the exhaust holes 61 located on the outer side of the exhaust assembly 60 is smaller than the area of the exhaust holes 61 located on the inner side of the exhaust assembly 60.

It is creatively found in the present disclosure that when the area of the exhaust holes 61 located on the outer side of the exhaust assembly 60 is the same or approximately same as the area of the exhaust holes 61 located on the inner side of the exhaust assembly 60, louder noise will be generated. Therefore, in the present disclosure, the exhaust holes 61 are set in a way that the area on the inner side is different from the area on the outer side, and thus, the technical effect of effectively lowering the noise is achieved.

Preferably, at most two exhaust holes are adjacent to each of the exhaust holes 61. In other words, the exhaust holes 61 in the present disclosure are only distributed in a structure of one circle, one layer, one row or one column to avoid noise increase caused by exhaust hole aggregation.

As mentioned above, the mask system also includes a ventilation assembly. The ventilation assembly includes a second main body on which a safety member 70 is disposed. The safety member 70 may be constructed on the oronasal cushion 1 as mentioned above, and may also be disposed on a frame assembly 50 or an elbow 500 of a ventilation pipe.

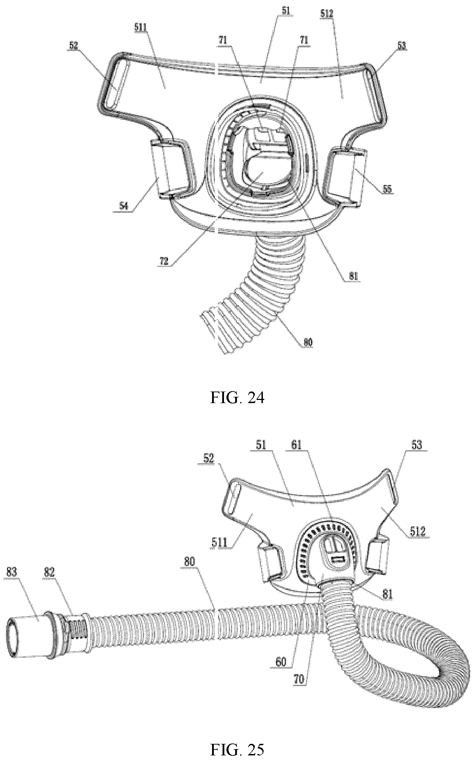

In a preferred implementation, the safety member 70 is constructed on the frame assembly 50, and the exhaust assembly 60 is also constructed on the frame assembly 50, as shown in FIG. 24 to FIG. 26 in combination with FIG. 16. Specifically, the safety member 70 includes a pipe body, a safety valve hole 71 disposed in a side wall of the pipe body and passing through the pipe body, and a safety valve plate 72, and the safety valve plate 72 is constructed to close or open the safety valve hole 71 according to a determination whether a pressure gas is introduced to the intake hole 62.

In the present implementation, the pipe body is constructed to be of a bent tubular structure, with one end being connected to the intake hole 62, and the other end being connected to a ventilation pipe by a pipe connecting part 73. In the present implementation, the ventilation pipe includes a flexible hose 80 with a first connecting end 81 being connected to the intake hole 62 of the frame 51, and a second connecting end 82 being connected to a pressure device by a connector 83.

The safety valve plate 72 is disposed in the safety member 70 and is hinged with an inner wall thereof. When a pressure gas is input to the safety member 70 through a ventilation pipe, the safety valve plate 72 upwards rotates to close the safety valve hole 71 under the drive of an airflow, at the moment, the ventilation pipe, the safety member 70, the intake hole 62 and the cavity 6 form a communicating passage to input a pressurized gas to the user.

When no gas is introduced, the safety valve plate 72 downwards rotates to open the safety valve hole 71, and thus, the safety valve hole 71 communicates with air. Therefore, when no gas is introduced to the mask system 400 or the pressure device to which the pressure gas is introduced is blocked or damaged to stop by accident, the safety valve hole 71 is opened by the safety valve plate 72, the safety valve hole 71 communicates with the air, and the user can inhale and exhale the air outside the mask system through the safety valve hole 71 to avoid the danger of asphyxia.

Further, when no gas is introduced, the safety valve plate 72 downwards rotates and covers one end of the pipe connecting part 73, and thus, the safety member 70 is disconnected to the ventilation pipe. Therefore, when no gas is introduced, if no gas is introduced to the mask system 400 or the pressure device is blocked or damaged to stop by accident, CO2 exhaled by the user cannot enter the pipe connecting part 73, either, and then cannot enter the intake hole 62 or the cavity 6, thereby preventing the user from repeatedly inhaling and exhaling CO2.

In the present implementation, optionally, the frame 51, the exhaust assembly 60 and the safety member 70 are individual parts and are connected together in a way such as mechanical connection, chemical binder or integrally molding. It can be understood that two or three of the frame 51, the exhaust assembly 60 and the safety member 70 are integrally formed; at the moment, the integrally formed components share a part of structure.

The second connecting end 82 of the flexible hose 80 is rotatably connected to the connector 83, and therefore, the connector 83 can rotate for three hundred and sixty degrees around the flexible hose 80.

The flexible hose 80 is usually a pipe of which the internal diameter is twelve millimeters to fifteen millimeters, and it may be an elastic pipe, such as a corrugated pipe of which the length is generally two hundred and fifty to four hundred and fifty millimeters. The flexible hose 80 is connected to the pressure device by an air delivery pipe. The air delivery pipe may have an internal diameter of fifteen millimeters to twenty-two millimeters, and a length of one thousand millimeters to two thousand millimeters.

The flexible hose 80 has the advantages that due to the better flexibility than the air delivery pipe, a dragging force generated by the air delivery pipe when a body moves can be overcome, and thus, the mask system is kept to be stably sealed on the face of the user.

In the present implementation, preferably, the frame 51, the exhaust assembly 60 and the safety member 70 are integrally formed.

Each of the frame 51, the exhaust assembly 60 and the safety member 70 may be made of a material such as PC, PP or ABS, etc. The frame 51, the exhaust assembly 60 and the safety member 70 may also be made of other thermoplastics.

In an embodiment shown in FIG. 27, the exhaust assembly 60 is respectively constructed on the frame 51 and the oronasal cushion 1, and the safety member 70 is constructed on the frame 51. In an embodiment shown in FIG. 28, the exhaust assembly 60 is constructed on the frame 51, and the safety member 70 is constructed on the oronasal cushion 1. The exhaust assembly 60 may be disposed in a way mentioned as the above-mentioned embodiments.

In a preferred implementation, the ventilation pipe includes an elbow 500 rotatably connected to the frame 51 and an elbow connector 600 connected to the elbow 500, and the elbow connector 600 is connected to the pressure device. Therefore, the present implementation differs from the above-mentioned implementation in that the elbow 500 may be rotatably connected to the frame 51.

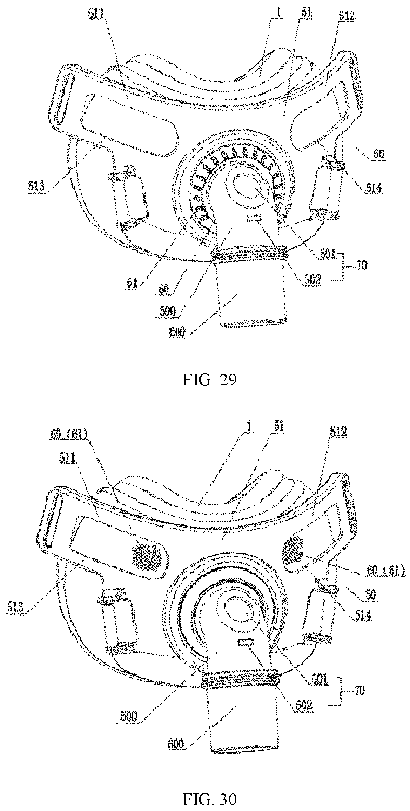

In the present embodiment, the safety member 70 is constructed on the elbow 500, as shown in FIG. 29 and FIG. 30. In an embodiment shown in FIG. 29, the exhaust assembly 60 is constructed on the frame 51, and the safety member 70 is constructed on the elbow 500. In an embodiment shown in FIG. 30, the exhaust assembly 60 is constructed on the oronasal cushion 1, and the safety member 70 is constructed on the elbow 500. The exhaust assembly 60 may be disposed in a way mentioned as the above-mentioned embodiments.

Specifically, the safety member 70 includes a safety valve hole 501 disposed in a side wall of the elbow 500 and passing through the elbow 500, and a safety valve plate 502, and the safety valve plate 502 is constructed to close or open the safety valve hole 501 according to a determination whether a pressure gas is introduced to the elbow 500.

The safety valve plate 502 is disposed in the elbow 500 and is hinged with an inner wall thereof. When a pressure gas is input to the elbow 500 through a ventilation pipe, the safety valve plate 502 upwards rotates to close the safety valve hole 501 under the drive of an airflow, at the moment, the ventilation pipe, the elbow 500, the intake hole 62 and the cavity 6 form a communicating passage to input a pressurized gas to the user.

When no gas is introduced, the safety valve plate 502 downwards rotates to open the safety valve hole 501, and thus, the safety valve hole 501 communicates with air. Therefore, when no gas is introduced to the mask system 400 or the pressure device to which the pressure gas is introduced is blocked or damaged to stop by accident, the safety valve hole 501 is opened by the safety valve plate 502, the safety valve hole 501 communicates with the air, and the user can inhale the air outside through the safety valve hole 501 to avoid the danger of asphyxia.

Further, when no gas is introduced, the safety valve plate 502 downwards rotates and completely covers the elbow 500 in the radial direction, and thus, the elbow 500 is disconnected to the ventilation pipe. Therefore, when no gas is introduced, if no gas is introduced to the mask system 400 or the pressure device is blocked or damaged to stop by accident, CO2 exhaled by the user cannot enter the elbow 500, either, and then cannot enter the intake hole 62 or the cavity 6, thereby preventing the user from repeatedly inhaling and exhaling CO2. A way of combination between the frame assembly 50 and the oronasal cushion 1 will be described in detail below. All of the above-mentioned embodiments and implementations may be combined with the embodiments described hereinafter.

With reference to FIG. 23, a cushion connecting part 58 is disposed on the frame 51, and the cushion connecting part 58 may be connected to the oronasal cushion 1 in a sealing way.

The cushion connecting part 58 is constructed as a boss on the inner side of the frame 51, a buckling part is disposed on an outer wall of the cushion connecting part 58, and the cushion connecting part 58 extends into the air inlet 41 and is matched with and connected to (in a buckling way) a sealing part 411 on an inner wall of the air inlet 41 (as shown in FIG. 11).

It can be understood that the cushion connecting part 58 may also be connected to the oronasal cushion 1 by a chemical binder.

In conclusion, according to the mask system 400 in the present disclosure, the exhaust holes 61 may be disposed in the frame 51, and the safety member 70 may be disposed on the frame 51, as shown in FIG. 16, FIG. 25 and FIG. 26.

Or the exhaust holes 61 are disposed in the oronasal cushion 1, and the safety member 70 is disposed on the frame 51, as shown in FIG. 27.

Or the exhaust holes 61 are disposed in the frame 51, and the safety member 70 (the safety valve hole 501 and the safety valve plate 502) is disposed on the oronasal cushion 1, as shown in FIG. 28.

Or the exhaust holes 61 are disposed in the frame 51, and the safety member 70 (the safety valve hole 501 and the safety valve plate 502) is disposed on the elbow 500, as shown in FIG. 29.

Or the exhaust holes 61 are disposed in the oronasal cushion 1, and the safety member 70 (the safety valve hole 501 and the safety valve plate 502) is disposed on the elbow 500, as shown in FIG. 30.

Or the exhaust holes 61 and the safety member 70 (the safety valve hole 501 and the safety valve plate 502) are respectively disposed on two sides of the oronasal cushion 1, and the elbow 500 is disposed, as shown in FIG. 31.

It can be known that positions where the exhaust holes 61 and the safety member 70 are disposed in the present disclosure may be combined arbitrarily, therefore, there are any other possible combinations which are not enumerated in the present disclosure.

A way of combination between the frame assembly 50 and the headband 90 will be described in detail below. All of the above-mentioned embodiments and implementations may be combined with the embodiments described hereinafter.

With reference to FIG. 16, the headband 90 includes upper headbands 91,92, lower headbands 94,95, and a top headband 93. The upper headbands 91,92 and the lower headbands 94,95 respectively extend to the back of the head along upsides and downsides of ears of the wearer, and the top headband 93 is placed on the top of the head of the user when being worn.

In an embodiment shown in FIG. 16, the two upper headbands 91,92 respectively extend from upsides of the left ear and the right ear of the wearer to the back of the head, the two lower headbands 95,94 respectively extend from downsides of the left ear and the right ear of the wearer to the back of the head, and thus, a back-of-head covering region 98 is formed; and the back-of-head covering region 98 connects the upper headbands 91,92 and the lower headbands 95,94 on the back of the head of the wearer so as to be capable of sharing a pressure of the upper headbands 91,92 and the lower headbands 95,94 on the head of the user.

In addition, in order to improve the wearing comfort level, the upper headbands 91,92 and the lower headbands 95,94 form an integer on the back of the head of the wearer, thereby preventing a connection from pressing the head of the wearer.

Further, the top headband 93 includes a first end band 931 and a second end band 932 respectively connected to the upper headbands 91,92, wherein a connecting piece is disposed on one of the first end band 931 and the second end band 932, a matching member is disposed on the other one of the first end band 931 and the second end band 932, and the matching member is connected to the connecting piece, so that the first end band 931 and the second end band 932 are connected on the top of the head of the wearer.

In the embodiment shown in FIG. 16, a connecting piece is disposed on the second end band 932, and the connecting piece is a headband buckle 934; and a matching member is disposed on the first end band 931, the matching member is a magic tape 933 which is reversely folded and stuck to its side part after passing through the headband buckle 934. Therefore, by adjusting a sticking position of the magic tape 933, the use length of the top headband 93 can be adjusted, that is, extension angles of the upper headbands 91,92 can be adjusted, and thus, a wearing force is adjusted.

The frame 51 in the above-mentioned embodiment has an approximately same integral structure. With an embodiment shown in FIG. 17 as an example, a first extension body 511 and a second extension body 512 extending obliquely and upwards are respectively disposed on two sides of the upper part of the frame 51, and the first extension body 511 and the second extension body 512 cover a part of the outer side of the oronasal cushion 1.

In an embodiment shown in FIG. 27 to FIG. 31, a first opening 513 and a second opening 514 are respectively disposed in the first extension body 511 and the second extension body 512, and the first opening 513 and the second opening 514 respectively extend in extension directions of the first extension body 511 and the second extension body 512.

In an embodiment shown in FIG. 27 and FIG. 30, the first opening 513 and the second opening 514 respectively correspond to the exhaust assembly 60 (wherein the exhaust assembly 60 is respectively located on two sides of the air inlet 41) integrally constructed on the oronasal cushion 1, and thus, the exhaust assembly 60 is exposed, which facilitates exhausting and reduces the weight of the frame 51.

In an embodiment shown in FIG. 28, the ventilation assembly (the safety member 70) and/or the exhaust assembly 60 is not constructed on the oronasal cushion 1, and therefore, the first opening 513 and the second opening 514 are only used to reduce the weight of the frame 51.

In an embodiment shown in FIG. 28, the first opening 513 and the second opening 514 respectively correspond to the ventilation assembly (the safety member 70) integrally constructed on the oronasal cushion 1, wherein the ventilation assembly (the safety member 70) is respectively located on two sides of the air inlet 41, and thus, the ventilation assembly is exposed, and the weight of the frame 51 can be reduced.

In an embodiment shown in FIG. 31, the first opening 513 corresponds to the ventilation assembly (the safety member 70) integrally constructed on the oronasal cushion 1, the second opening 514 corresponds to the exhaust assembly 60 integrally constructed on the oronasal cushion 1, wherein the ventilation assembly (the safety member 70) and the exhaust assembly 60 are respectively located on two sides of the air inlet 41, and thus, the ventilation assembly and the exhaust assembly 60 are exposed, and the weight of the frame 51 can be reduced.

With reference to FIG. 17, a first headband connecting hole 52 and a second headband connecting hole 53 are respectively disposed in the first extension body 511 and the second extension body 512, and a third headband connecting hole 54 and a fourth headband connecting hole 55 are respectively disposed in two sides of the lower part of the frame 51. The upper headbands 91,92 are respectively connected to the first headband connecting hole 52 and the second headband connecting hole 53, and the upper headbands 91,92 are constructed to extend to upsides of ears along the cheeks of the user in response to the headband 90 being worn by the user so as to adjust a pressure of the oronasal cushion on the nose of the user. The lower headbands 94,95 are respectively connected to the third headband connecting hole 54 and the fourth headband connecting hole 55, and the lower headbands 94,95 are constructed to extend to the back of the head of the user along two sides of a chin of the user in response to the headband 90 being worn by the user.

Each of the upper headbands 91,92 is provided with an adjusting mechanism, such as a magic tape, which is reversely folded and stuck to its side part after respectively passing through the first headband connecting hole 52 and the second headband connecting hole 53.

Optionally, the lower headbands 94,95 may be connected to the third headband connecting hole 54 and the fourth headband connecting hole 55 in the same way as the upper headbands 91,92.

Preferably, a first hasp 96 and a second hasp 97 are respectively disposed in the third headband connecting hole 54 and the fourth headband connecting hole 55, and the lower headbands 94,95 are connected to the third headband connecting hole 54 and the fourth headband connecting hole 55 through the first hasp 96 and the second hasp 97. The first hasp 96 and the second hasp 97 can expand the structure of the frame 51, can ensure more flexible connection between the headband 90 and the frame 51, and can reduce the cost.

In some optional implementations, as shown in FIG. 26, the frame 51 can also be connected to the headband 90 through a first bone beam arm 700 and a second bone beam arm 701. Specifically, the first bone beam arm 700 and the second bone beam arm 701 are respectively disposed on the first extension body 511 and the second extension body 512 on two sides of the upper part of the frame 51, wherein the first bone beam arm 700 and the second bone beam arm 701 respectively extend in extension directions of the first extension body 511 and the second extension body 512, the headband includes the upper headbands 91,92 and the lower headbands 94,95, and the upper headbands 91,92 are respectively connected to the first bone beam arm 700 and the second bone beam arm 701.

The first bone beam arm 700 and the second bone beam arm 701 can expand the structure of the frame 51, can improve the stability of the frame 51, and can reduce the cost.

A first bone beam arm connecting hole 702 and a second bone beam arm connecting hole 704 may be respectively disposed in ends of the first bone beam arm 700 and the second bone beam arm 701, and the upper headbands 91,92 are reversely folded and stuck to its outer side after respectively passing through the first bone beam arm connecting hole 702 and the second bone beam arm connecting hole 704.

The lower headbands 94,95 and the frame 51 may be constructed in one of the above-mentioned two ways, that is, the first extension body 511 and the second extension body 512 are only disposed, or the first bone beam arm 700 and the second bone beam arm 701 are respectively disposed on the first extension body 511 and the second extension body 512.

The thickness of the first bone beam arm 700 and the thickness of the second bone beam arm 701 are both zero point six to one point five millimeters, and are preferably zero point nine to one point two millimeters.

The first bone beam arm 700 and the second bone beam arm 701 may be both made of a material such as PC, PP and ABS, and may be also made of other thermoplastic materials.

The first bone beam arm 700 and the second bone beam arm 701 may be formed with the frame 51 by integral injection molding. Or, the first bone beam arm 700 and the second bone beam arm 701 may be connected to the frame 51 by adopting a mechanical buckling position or a chemical binder after being respectively formed. Especially, all technical features mentioned in all the embodiments can be combined in any way as long as there are no structural conflicts.

## Claims

1. An exhaust assembly for a mask system (400), comprising a first main body, the first main body comprising an intake hole (62) and a side wall (63) disposed around the circumferential direction of the intake hole (62), and exhaust holes (61) being disposed in the side wall; the intake hole (62) being configured to deliver a gas entering the intake hole (62) to a wearer wearing the mask system (400), and the exhaust holes (61) being configured to exhaust the gas exhaled by the wearer from the exhaust holes (61); and
an included angle a being formed between a normal plane (PI) in an intake direction in the intake hole (62) and the side wall,
**characterized in that** inner hole diameters of the exhaust holes (61) are greater than outer hole diameters of the exhaust holes (61).

2. The exhaust assembly according to claim 1, wherein the included angle a between the normal plane (PI) in the intake direction in the intake hole (62) and the side wall is ten degrees to seventy degrees.

3. The exhaust assembly according to claim 1 or 2, wherein an included angle ß is formed between the inner side wall of the exhaust holes (61) and the normal plane (PI);
the included angle ß between the inner side wall of the exhaust holes (61) and the normal plane (PI) is eighty degrees to one hundred degrees.

4. The exhaust assembly according to claim 1 or 2, wherein the average thickness of the side wall is zero point five millimeters to two point zero millimeters.

5. The exhaust assembly according to claim 1 or 2, wherein the exhaust holes (61) are constructed to ensure that a direction in which the gas enters the exhaust holes (61) and a direction in which the gas is exhausted from the exhaust holes (61) are parallel and non-collinear.

6. The exhaust assembly according to claim 1 or 2, wherein the exhaust holes (61) are annularly disposed in the circumferential direction of the intake hole (62); or
the exhaust holes (61) are distributed in a circle or ellipse concentric with the intake hole (62); or
the exhaust holes (61) are formed by up-and-down collision puncturing of molds.

7. The exhaust assembly according to claim 1 or 2, wherein the first main body is constructed on a frame assembly of the mask system (400), and forms a split structure or an integrated structure with the frame assembly of the mask system (400).

8. The exhaust assembly according to claim 1 or 2, wherein the first main body is constructed on a frame assembly (50) and a cushion assembly of the mask system (400), and respectively forms an integrated structure with the frame assembly (50) and the cushion assembly of the mask system (400); or
the first main body is constructed on a cushion assembly of the mask system (400), and forms a split structure or an integrated structure with the frame assembly (50) of the mask system (400).

9. A ventilation assembly for a mask system (400), comprising an exhaust assembly (60) according to claim 1, and a second main body, wherein a safety member (70) is disposed on the second main body, the safety member (70) is constructed to enable an intake hole (62) of the exhaust assembly (60) to communicate with a gas device when a gas is introduced to the mask system (400), and enable the mask system (400) to communicate with an environment when no gas is introduced to the mask system (400).

10. The ventilation assembly according to claim 9, wherein the second main body and the exhaust assembly (60) form the split structure, the exhaust assembly (60) is constructed on a frame assembly (50) of the mask system (400), and the second main body is constructed as a pipe body connected to the frame assembly (50).

11. The ventilation assembly according to claim 9, wherein the safety member (70) comprises a safety valve hole (501) and a safety valve plate, the safety valve hole (501) is connected to the intake hole (62), and the safety valve plate (502) is constructed to enable the intake hole (62) and the safety valve hole (501) not to be opened or closed at the same time.

12. The ventilation assembly according to claim 9, wherein the first main body and the second main body form the split structure, the first main body is constructed on a frame assembly (50) of the mask system (400), and the second main body is constructed on a cushion assembly of the mask system (400).

13. The ventilation assembly according to claim 9, wherein the first main body and the second main body form the integrated structure, and the first main body and the second main body are constructed on a frame assembly (50) or a cushion assembly of the mask system (400), or the first main body and the second main body form the split structure, the first main body is constructed on a frame assembly (50) of the mask system (400), and the second main body is constructed as an elbow connected to the frame assembly (50).

14. A frame assembly (50), comprising a frame on which the exhaust assembly (60) according to claim 1 and/or the ventilation assembly according to claim 9 are constructed.

## Patentansprüche

1. Auslassanordnung für ein Maskensystem (400), umfassend einen ersten Hauptkörper, wobei der erste Hauptkörper eine Einlassöffnung (62) und eine Seitenwand (63) umfasst, die um die Umfangsrichtung der Einlassöffnung (62) herum angeordnet ist, und Auslassöffnungen (61), die in der Seitenwand angeordnet sind; wobei die Einlassöffnung (62) dazu konfiguriert ist, ein in die Einlassöffnung (62) eintretendes Gas an einen Träger zu liefern, der das Maskensystem (400) trägt, und wobei die Auslassöffnungen (61) dazu konfiguriert sind, das von dem Träger ausgeatmete Gas aus den Auslassöffnungen (61) auszulassen; und
einen eingeschlossenen Winkel α, der zwischen einer Normalebene (PI) in einer Einlassrichtung in der Einlassöffnung (62) und der Seitenwand gebildet ist,
**dadurch gekennzeichnet, dass** die inneren Öffnungsdurchmesser der Auslassöffnungen (61) größer sind als die äußeren Öffnungsdurchmesser der Auslassöffnungen (61).

2. Auslassanordnung nach Anspruch 1, wobei der eingeschlossene Winkel α zwischen der Normalebene (PI) in der Einlassrichtung in der Einlassöffnung (62) und der Seitenwand zehn Grad bis siebzig Grad beträgt.

3. Auslassanordnung nach Anspruch 1 oder 2, wobei ein eingeschlossener Winkel β zwischen der inneren Seitenwand der Auslassöffnungen (61) und der Normalebene (PI) gebildet ist;
der eingeschlossene Winkel β zwischen der inneren Seitenwand der Auslassöffnungen (61) und der Normalebene (PI) achtzig Grad bis hundert Grad beträgt.

4. Auslassanordnung nach Anspruch 1 oder 2, wobei die durchschnittliche Dicke der Seitenwand null Komma fünf Millimeter bis zwei Komma null Millimeter beträgt.

5. Auslassanordnung nach Anspruch 1 oder 2, wobei die Auslassöffnungen (61) aufgebaut sind, um sicherzustellen, dass eine Richtung, in der das Gas in die Auslassöffnungen (61) eintritt, und eine Richtung, in der das Gas aus den Auslassöffnungen (61) austritt, parallel und nicht kollinear sind.

6. Auslassanordnung nach Anspruch 1 oder 2, wobei die Auslassöffnungen (61) ringförmig in der Umfangsrichtung der Einlassöffnung (62) angeordnet sind; oder
die Auslassöffnungen (61) in einem Kreis oder einer Ellipse verteilt sind, der/die konzentrisch zu der Einlassöffnung (62) ist; oder
die Auslassöffnungen (61) durch Auf- und Abwärts-Kollisionsdurchstoßen von Formen gebildet sind.

7. Auslassanordnung nach Anspruch 1 oder 2, wobei der erste Hauptkörper auf einer Rahmenanordnung des Maskensystems (400) aufgebaut ist und eine geteilte Struktur oder eine integrierte Struktur mit der Rahmenanordnung des Maskensystems (400) bildet.

8. Auslassanordnung nach Anspruch 1 oder 2, wobei der erste Hauptkörper auf einer Rahmenanordnung (50) und einer Polsteranordnung des Maskensystems (400) aufgebaut ist und jeweils eine integrierte Struktur mit der Rahmenanordnung (50) und der Polsteranordnung des Maskensystems (400) bildet; oder
der erste Hauptkörper auf einer Polsteranordnung des Maskensystems (400) aufgebaut ist und eine geteilte Struktur oder eine integrierte Struktur mit der Rahmenanordnung (50) des Maskensystems (400) bildet.

9. Belüftungsanordnung für ein Maskensystem (400), umfassend eine Auslassanordnung (60) nach Anspruch 1 und einen zweiten Hauptkörper, wobei ein Sicherheitselement (70) auf dem zweiten Hauptkörper angeordnet ist, das Sicherheitselement (70) aufgebaut ist, zu ermöglichen, dass eine Einlassöffnung (62) der Auslassanordnung (60) mit einer Gasvorrichtung kommuniziert, wenn ein Gas in das Maskensystem (400) eingeführt wird, und zu ermöglichen, dass das Maskensystem (400) mit einer Umgebung kommuniziert, wenn kein Gas in das Maskensystem (400) eingeführt wird.

10. Belüftungsanordnung nach Anspruch 9, wobei der zweite Hauptkörper und die Auslassanordnung (60) die geteilte Struktur bilden, die Auslassanordnung (60) auf einer Rahmenanordnung (50) des Maskensystems (400) aufgebaut ist und der zweite Hauptkörper als ein mit der Rahmenanordnung (50) verbundener Rohrkörper aufgebaut ist.

11. Belüftungsanordnung nach Anspruch 9, wobei das Sicherheitselement (70) eine Sicherheitsventilöffnung (501) und eine Sicherheitsventilplatte umfasst, die Sicherheitsventil-öffnung (501) mit der Einlassöffnung (62) verbunden ist und die Sicherheitsventilplatte (502) aufgebaut ist, zu ermöglichen, dass die Einlassöffnung (62) und die Sicherheitsventilöffnung (501) nicht gleichzeitig geöffnet oder geschlossen werden können.

12. Belüftungsanordnung nach Anspruch 9, wobei der erste Hauptkörper und der zweite Hauptkörper die geteilte Struktur bilden, der erste Hauptkörper auf einer Rahmenanordnung (50) des Maskensystems (400) aufgebaut ist und der zweite Hauptkörper auf einer Polsteranordnung des Maskensystems (400) aufgebaut ist.

13. Belüftungsanordnung nach Anspruch 9, wobei der erste Hauptkörper und der zweite Hauptkörper die integrierte Struktur bilden und der erste Hauptkörper und der zweite Hauptkörper auf einer Rahmenanordnung (50) oder einer Polsteranordnung des Maskensystems (400) aufgebaut sind, oder der erste Hauptkörper und der zweite Hauptkörper die geteilte Struktur bilden, der erste Hauptkörper auf einer Rahmenanordnung (50) des Maskensystems (400) aufgebaut ist und der zweite Hauptkörper als ein mit der Rahmenanordnung (50) verbundenes Knie aufgebaut ist.

14. Rahmenanordnung (50), umfassend einen Rahmen, auf dem die Auslassanordnung (60) nach Anspruch 1 und/oder die Belüftungsanordnung nach Anspruch 9 aufgebaut sind.

## Revendications

1. Ensemble d'évacuation pour un système type masque (400), comprenant un premier corps principal, le premier corps principal comprenant un trou d'entrée (62) et une paroi latérale (63) disposée autour de la direction circonférentielle du trou d'entrée (62), et des trous d'évacuation (61) étant disposés dans la paroi latérale ; le trou d'entrée (62) étant configuré pour délivrer un gaz entrant dans le trou d'entrée (62) vers un porteur portant le système type masque (400), et les trous d'évacuation (61) étant configurés pour l'évacuation du gaz expiré par le porteur depuis les trous d'évacuation (61) ; et
un angle inclus α étant formé entre un plan normal (PI) dans une direction d'entrée dans le trou d'entrée (62) et la paroi latérale,
**caractérisé en ce que** les diamètres de trou interne des trous d'évacuation (61) sont supérieurs aux diamètres de trou externe des trous d'évacuation (61).

2. Ensemble d'évacuation selon la revendication 1, dans lequel l'angle inclus α entre le plan normal (PI) dans la direction d'entrée du trou d'entrée (62) et la paroi latérale est de dix degrés à soixante-dix degrés.

3. Ensemble d'évacuation selon la revendication 1 ou 2, dans lequel un angle inclus β est formé entre la paroi latérale interne des trous d'évacuation (61) et le plan normal (PI) ;
l'angle inclus β entre la paroi latérale interne des trous d'évacuation (61) et le plan normal (PI) est de quatre-vingt degrés à cent degrés.

4. Ensemble d'évacuation selon la revendication 1 ou 2, dans lequel l'épaisseur moyenne de la paroi latérale est de zéro point cinq millimètre à deux point zéro millimètres.

5. Ensemble d'évacuation selon la revendication 1 ou 2, dans lequel les trous d'évacuation (61) sont construits pour garantir qu'une direction dans laquelle le gaz entre dans les trous d'évacuation (61) et une direction dans laquelle le gaz est évacué des trous d'évacuation (61) sont parallèles et non colinéaires.

6. Ensemble d'évacuation selon la revendication 1 ou 2, dans lequel les trous d'évacuation (61) sont disposés de manière annulaire dans la direction circonférentielle du trou d'entrée (62) ; ou
les trous d'évacuation (61) sont répartis selon un cercle ou une ellipse concentrique au trou d'entrée (62) ; ou
les trous d'évacuation (61) sont formés par une perforation par collision vers le haut et vers le bas des moules.

7. Ensemble d'évacuation selon la revendication 1 ou 2, dans lequel le premier corps principal est construit sur un ensemble formant cadre du système type masque (400), et forme une structure fendue ou une structure intégrée avec l'ensemble formant cadre du système type masque (400).

8. Ensemble d'évacuation selon la revendication 1 ou 2, dans lequel le premier corps principal est construit sur un ensemble formant cadre (50) et un ensemble formant coussinet du système type masque (400), et forme respectivement une structure intégrée avec l'ensemble formant cadre (50) et l'ensemble formant coussinet du système type masque (400) ; ou
le premier corps principal est construit sur un ensemble formant coussinet du système type masque (400), et forme une structure fendue ou une structure intégrée avec l'ensemble formant cadre (50) du système type masque (400).

9. Ensemble de ventilation pour un système type masque (400), comprenant un ensemble d'évacuation (60) selon la revendication 1, et un second corps principal, dans lequel un élément de sécurité (70) est disposé sur le second corps principal, l'élément de sécurité (70) est construit pour permettre à un trou d'entrée (62) de l'ensemble d'évacuation (60) de communiquer avec un dispositif de gaz lorsqu'un gaz est introduit au niveau du système type masque (400), et permettre au système type masque (400) de communiquer avec un environnement lorsqu'aucun gaz n'est introduit au niveau du système type masque (400).

10. Ensemble de ventilation selon la revendication 9, dans lequel le second corps principal et l'ensemble d'évacuation (60) forment la structure fendue, l'ensemble d'évacuation (60) est construit sur un ensemble formant cadre (50) du système type masque (400), et le second corps principal est construit sous la forme d'un corps de tuyau raccordé à l'ensemble formant cadre (50).

11. Ensemble de ventilation selon la revendication 9, dans lequel l'élément de sécurité (70) comprend un trou de soupape de sécurité (501) et une plaque de soupape de sécurité, le trou de soupape de sécurité (501) est raccordé au trou d'entrée (62), et la plaque de soupape de sécurité (502) est construite pour permettre de ne pas ouvrir ou fermer en même temps le trou d'entrée (62) et le trou de soupape de sécurité (501).

12. Ensemble de ventilation selon la revendication 9, dans lequel le premier corps principal et le second corps principal forment la structure fendue, le premier corps principal est construit sur un ensemble formant cadre (50) du système type masque (400), et le second corps principal est construit sur un ensemble formant coussinet du système type masque (400).

13. Ensemble de ventilation selon la revendication 9, dans lequel le premier corps principal et le second corps principal forment la structure intégrée, et le premier corps principal et le second corps principal sont construits sur un ensemble formant cadre (50) ou un ensemble formant coussinet du système type masque (400), ou le premier corps principal et le second corps principal forment la structure fendue, le premier corps principal est construit sur un ensemble formant cadre (50) du système type masque (400), et le second corps principal est construit comme un coude raccordé à l'ensemble formant cadre (50).

14. Ensemble formant cadre (50), comprenant un cadre sur lequel l'ensemble d'évacuation (60) selon la revendication 1 et/ou l'ensemble de ventilation selon la revendication 9 sont construits.
